# EUROPEAN PATENT SPECIFICATION

(11) **EP 2 064 916 B1**
(45) Date of publication and mention of the grant of the patent: **05.12.2018**
(21) Application number: 07841420.8
(22) Date of filing: 27.08.2007
(51) Int. Cl.: H04R 25/00, A61M 21/00, A61C 1/00

(54) **METHODS AND APPARATUS FOR TREATING TINNITUS**
VERFAHREN UND VORRICHTUNG ZUR TINNITUS-BEHANDLUNG
PROCÉDÉS ET APPAREIL DE TRAITEMENT DES ACOUPHÈNES

(30) Priority: 08.09.2006 US 825099 P
(43) Date of publication of application: 03.06.2009
(73) Proprietor: SoundMed, LLC, Mountain View, CA 94043 (US)
(72) Inventor: ABOLFATHI, Amir, Woodside, California 94062 (US); SPIRIDIGLIOZZI, John, San Mateo, California 94401 (US); KASSAYAN, Reza, Atherton, California 94027 (US)
(74) Representative: J A Kemp
(86) International application number: PCT/US2007/076912
(87) International publication number: WO 2008/030725

(56) References cited:
- WO-A2-2004/045242
- US-A- 5 447 489
- US-A- 5 800 336
- US-A- 5 800 336
- US-A1- 2005 020 873
- US-B1- 6 394 969

## Description

### FIELD OF THE INVENTION

The present invention relates to methods and apparatus for treating tinnitus via oral-based hearing aid appliances. More particularly, the present invention relates to methods and apparatus for treating tinnitus via oral appliances which are positionable within a mouth of a patient for transmitting sound conduction through teeth or bone structures in and/or around the mouth to mask or habituate a patient to sounds or ringing typically associated with tinnitus.

### BACKGROUND OF THE INVENTION

Tinnitus is a condition in which those affected perceive sound in one or both ears or in the head when no external sound is present. Often referred to as "ringing" in the ears, tinnitus can occur intermittently or consistently with a perceived volume ranging from low to painfully high. However, the perceived volume of tinnitus can vary from patient to patient where an objective measure of tinnitus volume in one patient may be perceived as painful but in another patient the same volume may be perceived as subtle.

Generally, tinnitus can be caused by a number of sources. For instance, exposure to loud noises can lead to damage of the cilia within the inner ear. An accumulation of wax within the ear canal can also amplify a person's tinnitus condition. Other factors such as ingestion of certain medications, ear or sinus infections, tumors growing on auditory nerves, as well as trauma to the head or neck can also induce tinnitus. Additionally, a small percentage of tinnitus patients may experience a form of tinnitus known as pulsatile tinnitus where a rhythmic pulsing sound is present which is attuned to the patient's heartbeat. Such a condition may be indicative of a cardiovascular condition such as pulmonary stenosis, hypertension, hardening of the arteries, arterio venous malformations, etc.

Treatments for tinnitus vary greatly. For instance, masking therapy typically involves using a hearing aid device to introduce sounds at a level and frequency that completely or partially cover the sounds of tinnitus in a patient to provide immediate short-term relief. Another similar therapy, tinnitus retraining therapy (TRT) or habituation, is a form of combination treatment that allows the patient to become comfortable with the tinnitus and defocuses their attention by utilizing sound generators such as hearing aids or even desktop devices such as fans to emit sounds at a lower level which still allow the user to hear the tinnitus with the intent of retraining the user's brain to eventually disregard the tinnitus. With habituation, a much lower level of sound therapy which does not mask the sound is delivered to the patient. In combination with therapy, habituation calms the patient and reinforces to them that their tinnitus is not life threatening or dangerous. Moreover, this therapy is meant to prevent the limbic system from increasing their awareness of and focus on Tinnitus. However, masking and TRT therapies may utilize conventional hearing aid devices which may be uncomfortable to the user and/or may carry other psychological stigmas. Additionally, in the case of TRT, such a therapy may take several years to accomplish.

Other devices such as cochlear implants and electrical stimulation, where an electrode array is inserted into the cochlea and a receiver is implanted subcutaneously behind the ear, may also be utilized to mask the tinnitus by ambient sounds and/or electrical stimulation. However, such procedures involve surgery and the complications typically associated therewith. Furthermore, drug therapy such as the use of antidepressants, may be effective in treating tinnitus. However, the typical side effects of ingesting such drugs may be highly undesirable to the tinnitus patient.

Accordingly, there exists a need for methods and devices for non-invasively and efficiently treating tinnitus patients.

US 5,800,336 discloses a floating mass transducer for assisting hearing in a person. Inertial vibration in the floating mass transducer produces vibrations in the inner ear. In an exemplary embodiment, the floating mass transducer comprises a magnet assembly and a coil secured inside a housing which is attached to bone within the middle ear. The coil is more rigidly secured to the housing than the magnet. The magnet assembly and coil are configured such that conducting alternating electrical current through the coil results in vibration of the magnet assembly and coil relative to one another. The vibration is caused by the interaction of the magnetic fields of the magnet assembly and coil. Because the coil is more rigidly secured to the housing than the magnet assembly, the vibrations of the coil cause the housing to vibrate. The floating mass transducer may generate vibrations in the inner ear by being attached to the skull or through a mouthpiece.

US 2003/0020873 discloses a totally implantable hearing prosthesis for hearing impaired persons. An inertial vibrational element is hermetically sealed and implanted in bone between the lateral and superior semicircular canals without breaching the integrity of the canals. The vibrational element is adapted to vibrate the walls of the canals and the fluids contained therein, thereby vibrating contiguous fluids within the cochlea thus stimulating hair cells and creating a hearing percept. The invention can also be adapted to be a tinnitus masking system, and/or used in combination with a coehlear implant hearing system.

### SUMMARY OF THE INVENTION

Tinnitus is a condition in which sound is perceived in one or both ears or in the head when no external sound is present. Such a condition may typically be treated by masking the tinnitus via a generated noise or sound.

According to the invention there are provided tinnitus treatment systems according to claims 1 and 9.

In one variation, the frequency or frequencies of the tinnitus may be determined through an audiology examination to pinpoint the range(s) in which the tinnitus occurs in the patient. This frequency or frequencies may then be programmed into a removable oral device which is configured
to generate sounds which are conducted via the user's tooth or bones to mask the tinnitus.

An electronic and transducer device may be attached, adhered, or otherwise embedded into or upon the removable oral appliance or other oral device to form a hearing aid and/or sound generating assembly. Such an oral appliance may be a custom-made device fabricated through a variety of different process utilizing, e.g., a replicate model of a dental structure obtained by any number of methods. The oral appliance may accordingly be created to fit, adhere, or be otherwise disposed upon a portion of the patient's dentition to maintain the electronics and transducer device against the patient's dentition securely and comfortably.

The electronic and transducer assembly may be programmed to generate sounds at one or more frequencies depending upon the condition of the user's tinnitus via a vibrating transducer element coupled to a tooth or other bone structure, such as the maxillary, mandibular, or palatine bone structure. Moreover, the assembly may also be optionally configured to receive incoming sounds either directly or through a receiver to process and amplify the signals and transmit the processed sounds. Sound (e.g. Any tone, music, or treatment using a wide-band or narrow-band noise) generated via an actuatable transducer is calibrated and equalized to compensate for impedances of the teeth and bone.

One method for treating tinnitus may generally comprise masking the tinnitus where at least one frequency of sound (e.g., any tone, music, or treatment using a wide-band or narrow-band noise) is generated via an actuatable transducer positioned against at least one tooth such that the sound is transmitted via vibratory conductance to an inner ear of the patient, whereby the sound completely or at least partially masks the tinnitus perceived by the patient. In generating a wide-band noise, the sound level may be raised to be at or above the tinnitus level to mask not only the perceived tinnitus but also other sounds. Alternatively, in generating a narrow-band noise, the sound level may be narrowed to the specific frequency of the tinnitus such that only the perceived tinnitus is masked and other frequencies of sound may still be perceived by the user.

Another method may treat the patient by habituating the patient to their tinnitus where the actuatable transducer may be vibrated within a wide-band or narrow-band noise targeted to the tinnitus frequency perceived by the patient overlayed upon a wide-frequency spectrum sound. This wide-frequency spectrum sound, e.g., music, may extend over a range which allows the patient to periodically hear their tinnitus through the sound and thus defocus their attention to the tinnitus.

In enhancing the treatment for tinnitus, a technician, audiologist, physician, etc., may first determine the one or more frequencies of tinnitus perceived by the patient. Once the one or more frequencies have been determined, the audiologist or physician may determine the type of treatment to be implemented, e.g., masking or habituation. Then this information may be utilized to develop the appropriate treatment and to compile the electronic treatment program file which may be transmitted, e.g., wirelessly, to a processor coupled to the actuatable transducer such that the transducer is programmed to vibrate in accordance with the treatment program.

In use, an oral appliance containing the transducer may be placed against one or more teeth of the patient and the transducer may be actuated by the user when tinnitus is perceived to generate the one or more frequencies against the tooth or teeth. The generated vibration may be transmitted via vibratory conductance through the tooth or teeth and to the inner ear of the patient such that each of the frequencies of the perceived tinnitus is masked completely or at least partially.

The oral appliance may be programmed with a tinnitus treatment algorithm which utilizes the one or more frequencies for treatment. This tinnitus treatment algorithm may be uploaded to the oral appliance wirelessly by an external programming device to enable the actuator to vibrate according to the algorithm for treating the tinnitus. Moreover, the oral appliance may be used alone for treating tinnitus or in combination with one or more hearing aid devices for treating patients who suffer not only from tinnitus but also from hearing loss.

### BRIEF DESCRIPTION OF THE DRAWINGS

Fig. 1 illustrates the dentition of a patient's teeth and one variation of a hearing aid and/or sound generating assembly which is removably placed upon or against the patient's tooth or teeth as a removable oral appliance.
Fig. 2A illustrates a perspective view of the lower teeth showing one exemplary location for placement of the removable oral appliance hearing aid and/or sound generating assembly.
Fig. 2B illustrates another variation of the removable oral appliance in the form of an appliance which is placed over an entire row of teeth in the manner of a mouthguard.
Fig. 2C illustrates another variation of the removable oral appliance which is supported by an arch.
Fig. 2D illustrates another variation of an oral appliance configured as a mouthguard.
Fig. 3 illustrates a detail perspective view of the oral appliance positioned upon the patient's teeth utilizable in combination with a transmitting assembly external to the mouth and wearable by the patient in another variation of the device.
Fig. 4 shows an illustrative configuration of the individual components in a variation of the oral appliance device having an external transmitting assembly with a receiving and transducer assembly within the mouth.
Fig. 5 shows an illustrative configuration of another variation of the device in which the entire assembly is contained by the oral appliance within the user's mouth.
Fig. 6A shows yet another illustrative variation of the device in which the sound generating device may be connected to a receiver for receiving programming signals to treat patient-specific tinnitus conditions.
Fig. 6B shows an example where the device assembly may be actuated via a separate transmitter assembly to control the operation of the device.
Fig. 7 illustrates a variation of one method for obtaining frequencies associated with tinnitus and which are patient-specific for programming an oral appliance.
Fig. 8A illustrates several variations for programming the electronics and/or transducer assembly with patient-specific tinnitus frequency or frequencies.
Fig. 8B schematically illustrates a variation where the electronics are separated from the transducer assembly.
Figs. 9A and 9B illustrate examples for automatically decreasing the amplification level of generated sounds for treating tinnitus.
Figs. 10A and 10B illustrate additional examples for automatically decreasing or terminating generated sounds for treating tinnitus.
Fig. 11 illustrates another example for decreasing generated sounds over a period of days, weeks, months, etc. to facilitate the withdrawal of a user's dependence on the masking noise.
Figs. 12A and 12B illustrate an example for transmitting and correlating a patient's heart rate to masking sounds generated by an oral appliance.
Figs. 13A and 13B illustrate top views of variations of a retainer integrated with one or more transducer assemblies.
Fig. 14A shows an example of a tinnitus treatment system using an external device for wirelessly transmitting a tinnitus treatment algorithm to a retainer assembly.
Fig. 14B shows another example of a tinnitus treatment system using the retainer assembly in conjunction with a hearing aid device.
Fig. 15 shows another example of a combination therapy system utilizing a microphone and a tinnitus treatment algorithm for transmission of auditory signals and treatment algorithm to the retainer assembly.

### DETAILED DESCRIPTION OF THE INVENTION

Because tinnitus is a condition in which sound is perceived in one or both ears or in the head when no external sound is present, such a condition may typically be treated by masking the tinnitus via a generated noise or sound. In one variation, the frequency or frequencies of the tinnitus may be determined through an audiology examination to pinpoint the range(s) in which the tinnitus occurs in the patient. This frequency or frequencies may then be programmed into a removable oral device which is configured to generate sounds which are conducted via the user's tooth or bones to mask the tinnitus, as described in further detail below.

An electronic and transducer device may be attached, adhered, or otherwise embedded into or upon the removable oral appliance or other oral device to form a hearing aid and/or sound generating assembly. Such an oral appliance may be a custom-made device fabricated through a variety of different process utilizing, e.g., a replicate model of a dental structure obtained by any number of methods. The oral appliance may accordingly be created to fit, adhere, or be otherwise disposed upon a portion of the patient's dentition to maintain the electronics and transducer device against the patient's dentition securely and comfortably.

The electronic and transducer assembly may be programmed to generate sounds at one or more frequencies depending upon the condition of the user's tinnitus via a vibrating transducer element coupled to a tooth or other bone structure, such as the maxillary, mandibular, or palatine bone structure. Moreover, the assembly may also be optionally configured to receive incoming sounds either directly or through a receiver to process and amplify the signals and transmit the processed sounds. Any tone, music, or treatment using a wide-band and or narrow band noise is calibrated and equalized to compensate for impedances of the tooth and bone and then that sound is generated via the actuatable transducer. Calibration and equalization can be done using several approaches. One approach is to use average impedance among a group of subjects representative of the targeted population. Another approach is to customize the calibration and equalization by obtaining the teeth and bone impedances for each patient.

Moreover, the electronic and transducer assembly may be configured to provide several different tinnitus treatments. For instance, the assembly may be configured to provide tinnitus masking therapy by providing sounds through bone conduction at a level and frequency that completely or partially cover the sounds of tinnitus to provide immediate short-term relief. Any tone, music, or treatment using a wide-band or narrow-band noise may be generated via the actuatable transducer positioned against at least one tooth such that the sound is transmitted via vibratory conductance to an inner ear of the patient, whereby the sound completely or at least partially masks the tinnitus perceived by the patient.

Alternatively, the assembly may be configured to provide habituation treatment, where the assembly provides sounds which may not mask the tinnitus but allows the patient to defocus their attention. The actuatable transducer may be vibrated within a wide-band or narrow-band noise targeted to the tinnitus frequency perceived by the patient overlayed upon a wide-frequency spectrum sound. This wide-frequency spectrum sound, e.g., music, may extend over a range which allows the patient to periodically hear their tinnitus through the sound and thus defocus their attention to the tinnitus.

Typically, this involves having a patient or treatment provider select a pleasant monaural piece of music having large fluctuations. The level fluctuations are preferably chosen to allow for the intermittent perception of the tinnitus by the patient, i.e., the tinnitus may be perceived by the patient during quiet passages in the music. A broadband, e.g., 14 kHz, white noise may be added or overlayed upon the music at a level that just masks the tinnitus yet still allows the music to be heard. The treatment provider may add amplification to the music and/or broadband white noise, e.g., via a graphic equalizer, to compensate for any hearing loss by the patient.

Taking this music and overlayed broadband white noise, an electronic stereo file may be produced from the monaural file where the same monaural file is used in each channel to equalize the phase. This treatment file may then be played by the patient, e.g., through an electronic music player and/or transmitted through the transducer.

In any of the treatment mechanisms or devices, either masking or habituation treatment may be effected by the assemblies described herein.

In yet another tinnitus treatment method similar to acoustic echo cancellation, an audiologist or physician may determine the tinnitus frequency perceived by a patient. With the frequency or frequencies known, a treatment signal may be generated, e.g., 5 kHz at 6 dB, which is shifted out-of-phase from the tinnitus frequencies, e.g., ideally 180° out-of-phase. This shifted treatment signal may be transmitted to a processor which actuates the transducer to vibrate the out-of-phase treatment signal through the patient's tooth, teeth, or bone structures such that the summation of the treatment signal with the tinnitus results in a cancellation of the tinnitus noise as perceived by the patient. Examples and further details of signal cancellation methods are described in U.S. Pat. App. 11/672,239 filed February 7, 2007, which is incorporated herein by reference in its entirety.

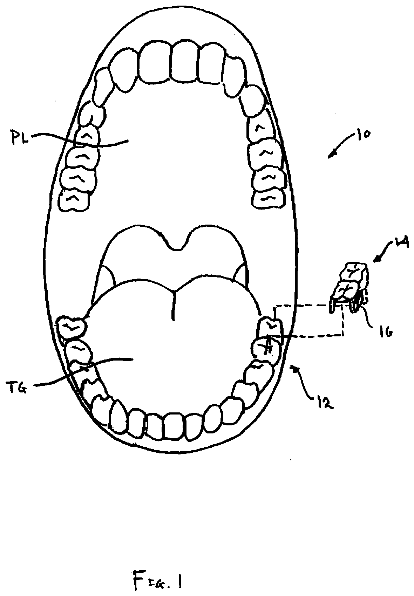

As shown in Fig. 1, a patient's mouth and dentition **10** is illustrated showing one possible location for removably attaching hearing aid and/or sound generating assembly **14** upon or against at least one tooth, such as a molar **12.** The patient's tongue **TG** and palate **PL** are also illustrated for reference. An electronics and/or transducer assembly **16** may be attached, adhered, or otherwise embedded into or upon the assembly **14,** as described below in further detail.

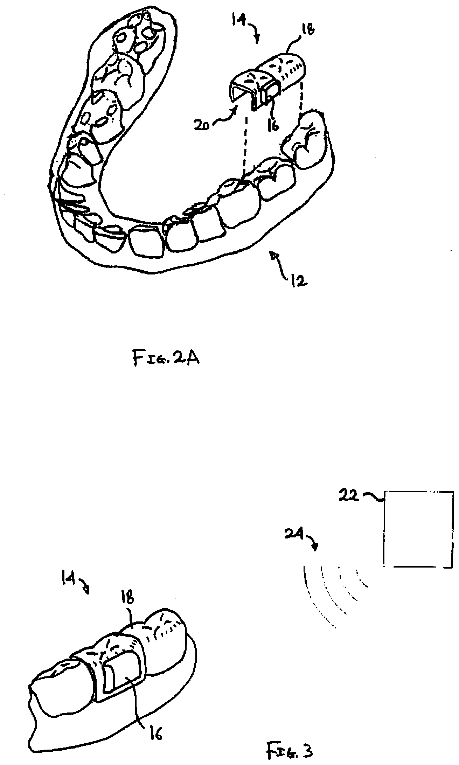

Fig. 2A shows a perspective view of the patient's lower dentition illustrating the hearing aid and/or sound generating assembly **14** comprising a removable oral appliance **18** and the electronics and/or transducer assembly **16** positioned along a side surface of the assembly **14.** In this variation, oral appliance **18** may be fitted upon two molars **12** within tooth engaging channel **20** defined by oral appliance **18** for stability upon the patient's teeth, although in other variations, a single molar or tooth may be utilized. Alternatively, more than two molars may be utilized for the oral appliance **18** to be attached upon or over. Moreover, electronics and/or transducer assembly **16** is shown positioned upon a side surface of oral appliance **18** such that the assembly **16** is aligned along a buccal surface of the tooth **12;** however, other surfaces such as the lingual surface of the tooth **12** and other positions may also be utilized. The figures are illustrative of variations and are not intended to be limiting; accordingly, other configurations and shapes for oral appliance **18** are intended to be included herein.

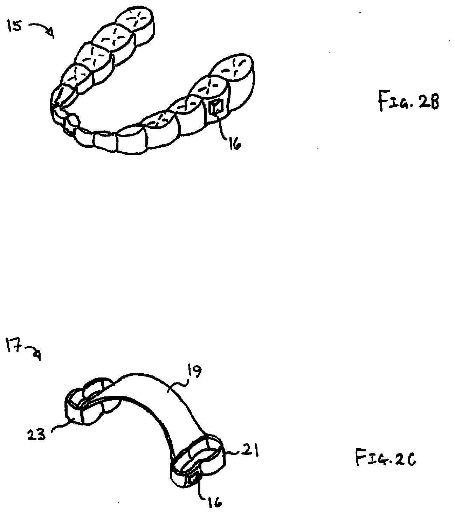

Fig. 2B shows another variation of a removable oral appliance in the form of an appliance **15** which is placed over an entire row of teeth in the manner of a mouthguard. In this variation, appliance **15** may be configured to cover an entire bottom row of teeth or alternatively an entire upper row of teeth. In additional variations, rather than covering the entire rows of teeth, a majority of the row of teeth may be instead be covered by appliance **15.** Assembly **16** may be positioned along one or more portions of the oral appliance **15.**

Fig. 2C shows yet another variation of an oral appliance **17** having an arched configuration. In this appliance, one or more tooth retaining portions **21, 23,** which in this variation may be placed along the upper row of teeth, may be supported by an arch **19** which may lie adjacent or along the palate of the user. As shown, electronics and/or transducer assembly **16** may be positioned along one or more portions of the tooth retaining portions **21, 23.** Moreover, although the variation shown illustrates an arch **19** which may cover only a portion of the palate of the user, other variations may be configured to have an arch which covers the entire palate of the user.

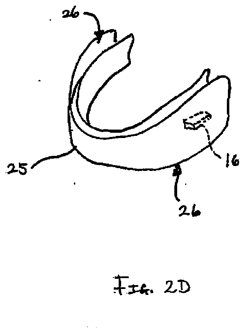

Fig. 2D illustrates yet another variation of an oral appliance in the form of a mouthguard or retainer **25** which may be inserted and removed easily from the user's mouth. Such a mouthguard or retainer **25** may be used in sports where conventional mouthguards are worn; however, mouthguard or retainer **25** having assembly **16** integrated therein may be utilized by persons, hearing impaired or otherwise, who may simply hold the mouthguard or retainer **25** via grooves or channels **26** between their teeth for receiving instructions remotely and communicating over a distance.

Generally, the volume of electronics and/or transducer assembly **16** may be minimized so as to be unobtrusive and as comfortable to the user when placed in the mouth. Although the size may be varied, a volume of assembly **16** may be less than 800 cubic millimeters. This volume is, of course, illustrative and not limiting as size and volume of assembly **16** and may be varied accordingly between different users.

In one variation configured as a hearing aid device, with assembly **14** positioned upon the teeth, as shown in Fig. 3, an extra-buccal transmitter assembly **22** located outside the patient's mouth may be utilized to receive auditory signals for processing and transmission via a wireless signal **24** to the electronics and/or transducer assembly **16** positioned within the patient's mouth, which may then process and transmit the processed auditory signals via vibratory conductance to the underlying tooth and consequently to the patient's inner ear.

The transmitter assembly **22,** as described in further detail below, may contain a microphone assembly as well as a transmitter assembly and may be configured in any number of shapes and forms worn by the user, such as a watch, necklace, lapel, phone, belt-mounted device, etc.

Alternatively in another variation, transmitter assembly **22** may be configured as a transmitter for sending programming signals to electronics and/or transducer assembly **16** for programming specified frequencies or duration times for the transducer to vibrate, as described in further detail below.

In either case, in this and other variations, the transducer assembly **16** may generally be configured to have a frequency response of, e.g., 125 Hz to 20 kHz at 100 dB sound pressure level (SPL) peak and a frequency response of, e.g., 125 Hz to 1000 Hz based on uncomfortable vibration (UCV).

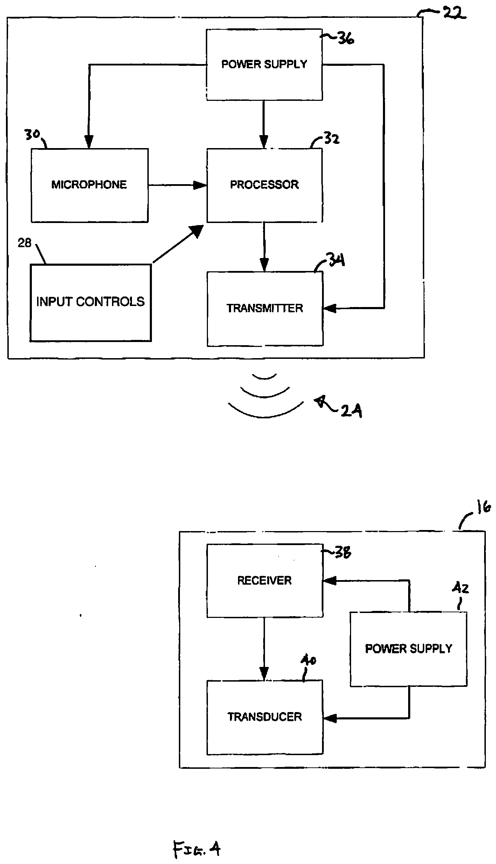

Fig. 4 illustrates a schematic representation of the variation where assembly **14** is configured as a hearing aid device utilizing an extra-buccal transmitter assembly **22,** which may generally comprise microphone **30** for receiving sounds and which is electrically connected to processor **32** for processing the auditory signals. Processor **32** may be connected electrically to transmitter **34** for transmitting the processed signals to the electronics and/or transducer assembly **16** disposed upon or adjacent to the user's teeth. The microphone **30** and processor **32** may be configured to detect and process auditory signals in any practicable range, but may be configured in one variation to detect auditory signals ranging from, e.g., 125 Hertz to 20,000 Hertz.

With respect to microphone **30,** a variety of various microphone systems may be utilized. For instance, microphone **30** may be a digital, analog, and/or directional type microphone. Such various types of microphones may be interchangeably configured to be utilized with the assembly, if so desired.

Power supply **36** may be connected to each of the components in transmitter assembly **22** to provide power thereto. The transmitter signals **24** may be in any wireless form utilizing, e.g., radio frequency, ultrasound, microwave, Blue Tooth® (BLUETOOTH SIG, INC., Bellevue, WA), etc. for transmission to assembly **16.** Assembly **22** may also optionally include one or more input controls **28** that a user may manipulate to adjust various acoustic parameters of the electronics and/or transducer assembly **16,** such as acoustic focusing, volume control, filtration, muting, frequency optimization, sound adjustments, and tone adjustments, etc.

The signals transmitted **24** by transmitter **34** may be received by electronics and/or transducer assembly **16** via receiver **38,** which may be connected to an internal processor for additional processing of the received signals. The received signals may be communicated to transducer **40,** which may vibrate correspondingly against a surface of the tooth to conduct the vibratory signals through the tooth and bone and subsequently to the middle ear to facilitate hearing of the user. Transducer **40** may be configured as any number of different vibratory mechanisms. For instance, in one variation, transducer **40** may be an electromagnetically actuated transducer. In other variations, transducer **40** may be in the form of a piezoelectric crystal having a range of vibratory frequencies, e.g., between 250 Hz to 14,000 Hz.

Power supply **42** may also be included with assembly **16** to provide power to the receiver, transducer, and/or processor, if also included. Although power supply **42** may be a simple battery, replaceable or permanent, other variations may include a power supply **42** which is charged by inductance via an external charger, e.g., every 24 hours. Additionally, power supply **42** may alternatively be charged via direct coupling to an alternating current (AC) or direct current (DC) source. Other variations may include a power supply **42** which is charged via a mechanical mechanism, such as an internal pendulum or slidable electrical inductance charger as known in the art, which is actuated via, e.g., motions of the jaw and/or movement for translating the mechanical motion into stored electrical energy for charging power supply **42.** Moreover, the power supply **42** may be disposable where either the power supply **42** itself (if removable) or the entire assembly **16** may be disposed and replaced by a new assembly periodically, e.g., every 4 weeks.

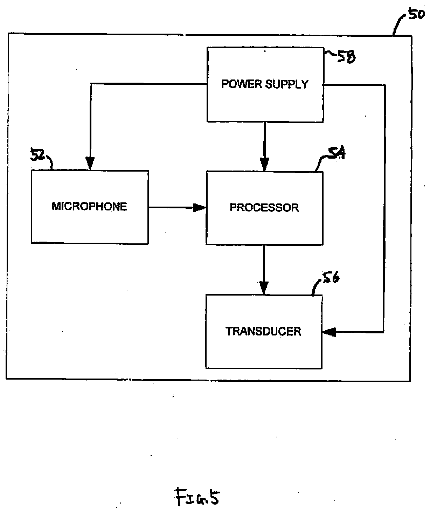

In another variation of assembly **16,** rather than utilizing an extra-buccal transmitter, hearing aid assembly **50** may be configured as an independent assembly contained entirely within the user's mouth, as shown in Fig. 5. Accordingly, assembly **50** may include an internal microphone **52** in communication with an on-board processor **54.** Internal microphone **52** may comprise any number of different types of microphones, as described above. Processor **54** may be used to process any received auditory signals for filtering and/or amplifying the signals and transmitting them to transducer **56,** which is in vibratory contact against the tooth surface. Power supply **58,** as described above, may also be included within assembly **50** for providing power to each of the components of assembly **50** as necessary.

The removable oral appliance **18** may be fabricated from various polymeric or a combination of polymeric and metallic materials using any variety of methods. For instance, in one variation of fabricating an oral appliance, a three-dimensional digital scanner may be used to image the dentition of the patient, particularly the tooth or teeth upon or about which the oral appliance is to be positioned. The scanned image may be processed via a computer to create a three-dimensional virtual or digital model of the tooth or teeth.

Various three-dimensional scanning modalities may be utilized to create the three-dimensional digital model. For instance, intra-oral cameras or scanners using, e.g., laser, white light, ultrasound, mechanical three-dimensional touch scanners, magnetic resonance imaging (MRI), computed tomography (CT), other optical methods, etc., may be utilized.

Once the three-dimensional image has been captured, the image may then be manipulated via conventional software to create a direct three-dimensional print of the model. Alternatively, the image may be used to directly machine the model. Systems such as computer numerical control (CNC) systems or three-dimensional printing processes, e.g., stereolithography apparatus (SLA), selective laser sintering (SLS), and/or other similar processes utilizing three-dimensional geometry of the patient's dentition may be utilized.

In another alternative, a mold may be generated from the print to then allow for thermal forming of the appliance directly upon the created mold. And yet in other variations, the three-dimensional image may be used to create an injection mold for creating the appliance.

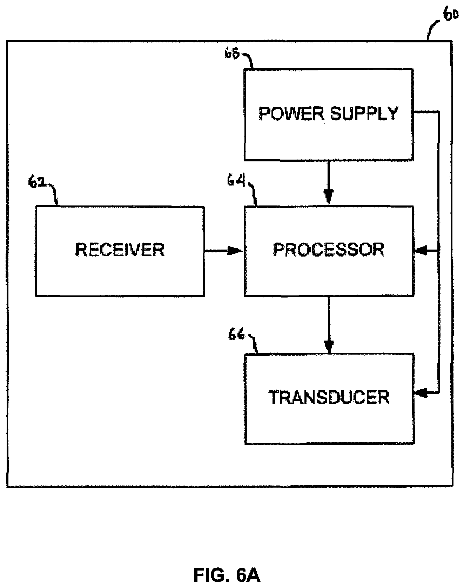

In another variation of the device configured to additionally treat tinnitus instead of or in combination with treating hearing loss, sound generating assembly **60** may optionally contain a receiver **62** for receiving programming signals **24** from transmitter **34.** Receiver **62** may be in electrical communication with processor **64,** powered by power supply **68,** which in turn is electrically coupled to transducer **66,** as shown in the schematic representation of Fig. 6A.

Power supply **68** may provide power to the receiver **62,** transducer **66,** and/or processor **64.** Although power supply **68** may be a simple battery, replaceable or permanent, other variations may include a power supply **68** which is charged by inductance via an external charger. Additionally, power supply **68** may alternatively be charged via direct coupling to an alternating current (AC) or direct current (DC) source. Other variations may include a power supply **68** which is charged via a mechanical mechanism, such as an internal pendulum or slidable electrical inductance charger as known in the art, which is actuated via, e.g., motions of the jaw and/or movement for translating the mechanical motion into stored electrical energy for charging power supply **68.**

In the variation where the sound generating assembly **60** is configured to function solely as a sound generating device to mask tinnitus, receiver **62** may be omitted from assembly **60** and transducer **66** may be configured to vibrate at a predetermined frequency or over a range of predetermined frequencies, e.g., anywhere from 250 Hz to 14,000 Hz, for a predetermined period of time, e.g., on the order of a few minutes up to several hours, as desired. The assembly may be accordingly actuated by the user on demand when desired to mask the tinnitus such that the transducer **66** vibrates, e.g., anywhere from 250 Hz to 14,000 Hz, for a specified preset time period or until deactivated by the user.

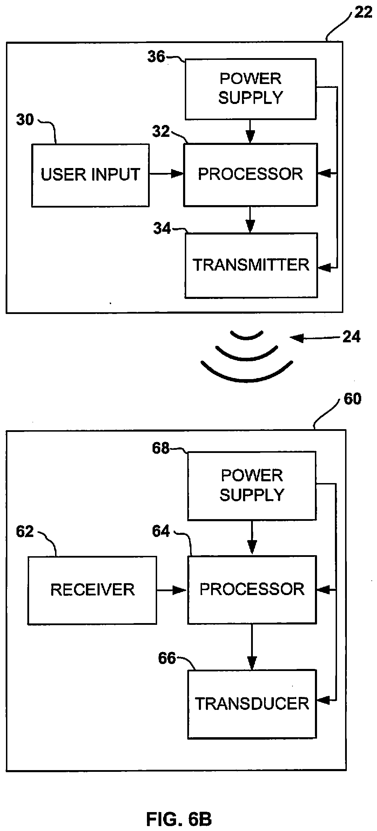

In the variation illustrated in Fig. 6B, assembly **60** may be actuated via transmitter assembly **22,** as described above, to control the operation of the assembly **60.** The transmitter signals **24** may be in any wireless form utilizing, e.g., radio frequency, ultrasound, microwave, Blue Tooth® (BLUETOOTH SIG, INC., Bellevue, WA), etc. for transmission to assembly **60.** Assembly **22** may also optionally include one or more input controls **30** that a user may manipulate to turn the assembly **60** on or off as well as to optionally adjust various acoustic parameters of the electronics and/or transducer assembly **16,** such as acoustic focusing, volume control, filtration, muting, frequency optimization and/or selection, sound adjustments, tone adjustments, time of operation or time delay of the transducer, etc.

Additionally, user input controls **30** may also include a feature to program and control the automatic activation or de-activation of the transducer **66** at preset times throughout the day, e.g., such as an alarm feature to automatically awake the user at a selected time or to automatically activate the transducer **66** at a selected time prior to or during the user's bedtime to automatically mask completely or partially the tinnitus.

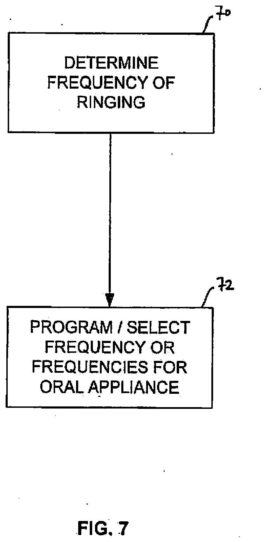

In an alternative variation, the assembly **60** may be configured to receive programming signals received by receiver **62.** In such a variation, the device may be specifically programmed to vibrate the transducer **66** at specified frequencies and/or for specified periods of time which may be customized to patient-specific tinnitus conditions. Accordingly, the patient may be examined, e.g., by a technician, audiologist, physician, etc., to initially determine the frequency or frequencies of the tinnitus perceived by the patient **70,** as indicated in Fig. 7, utilizing any audiology instruments or procedures such as tuning forks, audiometry, etc.

Once the patient-specific tinnitus frequency or frequencies have been determined, these frequency values may be programmed for an oral appliance **72** such that the transducer **66** may vibrate at the specified frequency or frequencies to optimally mask, or at least partially mask, the tinnitus. Alternatively, if the detected frequency or frequencies of tinnitus fall within certain frequency ranges, the oral appliance assembly **60** may be configured simply to vibrate the transducer **66** within preset frequency ranges rather than specific targeted frequency values.

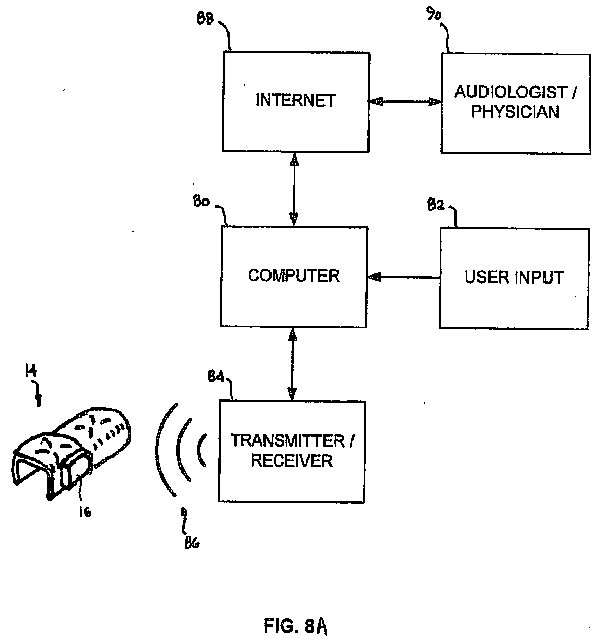

In order to program the electronics and/or transducer assembly **16** with patient-specific tinnitus frequency or frequencies, several alternative methods may be utilized to appropriately program the assembly **16,** as illustrated in Fig. 8A. For instance, a technician, audiologist, physician, etc. may directly program the assembly **16** with a computer **80** in communication with a transmitter **84** to wirelessly transmit programming information **86** to receiver **62** contained within assembly **16.**

Alternatively, a user may directly input **82** patient-related frequency information via a computer **80** to transmit the programming information **86** to assembly **16** via transmitter **84.** In yet another variation, computer **80** may be connected to the internet **88** through which a technician, audiologist, physician, etc. **90** may input and/or access patient-specific frequency information for transmission to computer **80,** which may then be used to transmit the information via transmitter **84** to assembly **16.** Transmitter **84** may also be utilized as a receiver to optionally receive patient-specific information from assembly **16**, in which case a transmitter may be incorporated into assembly **16.**

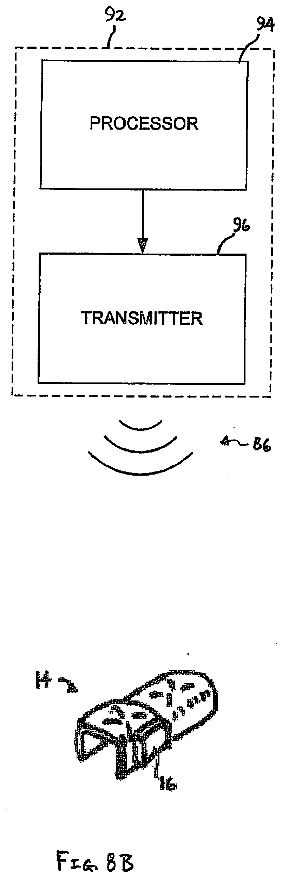

In another variation for treating tinnitus, the electronics may be separated from the transducer assembly **16** to provide for a potentially smaller and less intrusive device **14** for delivering a masking treatment to the patient. As schematically illustrated in Fig. 8B, a base unit **92** may incorporate the electronics, including at least processor **94** and transmitter **96,** to wirelessly transmit programming information **86** to the transducer assembly **16** for conductance to the patient. Base unit **92** may be configured into any number of different form factors, such as a base unit for placement on a nightstand or tabletop. Alternatively, base unit **92** may be configured for attachment onto a patient's belt much like a music player or IPOD device (Apple, Inc., Cupertino, CA). The transducer assembly **16** may contain a receiver for receiving the tinnitus masking or therapy programming information **86,** a transducer for conducting the signals to the patient, and a power supply, as described above. In this and other variations where the transducer assembly **16** is configured to provide tinnitus habituation treatment, the programming information **86** may be combined or overlayed with music as selected by the user. Because other electronic components may be contained within base unit **92** rather than assembly **16,** the device **14** may be configured into a relatively smaller configuration.

In other variations, rather than utilizing a device **14** which is placed within the mouth of a patient, assembly **16** may comprise an adhesive-backed assembly which may be temporarily attached at the entrance to the patient's ear canal and removed after use and disposed. In either case, the assembly **16** may be used by the patient at night prior to sleeping where base unit **92** may generate and wirelessly transmit the programming to the patient via device **14.**

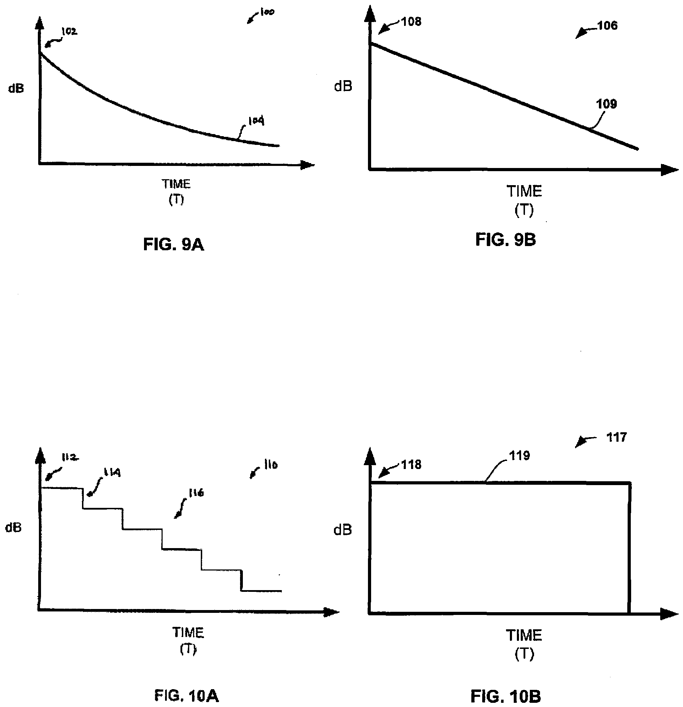

Aside from the ability to program specific frequencies into assembly **16** for which to vibrate the transducer **66,** other patient-specific information such as periods of time for vibrating may also be programmed. Moreover, the amplification of the generated sound may also be eventually decreased automatically over this period of time in order to gradually decrease the user's dependence on the device, e.g., prior to and during the initial phases of sleep. In one variation, as shown in Fig. 9A, the transducer **66** may be programmed to vibrate at one or more specified frequencies upon actuation at a first starting decibel (dB) level **102,** e.g., 30 dB to 40 dB. The first starting level **102** may be varied depending upon the user's tinnitus condition. Over a specified time period, **T,** e.g., anywhere from several minutes to several hours, the amplification level of the vibrating transducer **66** may be reduced exponentially **104,** as shown in the plot **100** to gradually reduce the masking noise until the transducer **66** is stopped altogether. Alternatively, the amplification level may be reduced linearly, as shown in the plot **106** in Fig. 9B, such that the transducer **66** may begin at a first starting decibel level **108** upon actuation and gradually reduces linearly **109** until the transducer **66** is stopped altogether.

In these and other examples, although the levels are illustrated as decreasing over time, they may alternatively be increased for set periods of time intermittently or gradually over time, depending upon the desired treatment.

In another alternative, Fig. 10A illustrates a plot **110** where the amplification level may be stepped **116** such that the level begins at a first constant decibel level **112** and then after a period of time, **T,** steps down **114** to a second level. The amplification may be eventually stepped down in uniform (or non-uniform) increments until the transducer **66** is stopped altogether. In another variation, the amplification may begin at a first level **118** and maintain a constant level **119** for a period of time, **T,** until the device is stopped altogether, as shown in the plot **117** in Fig. 10B.

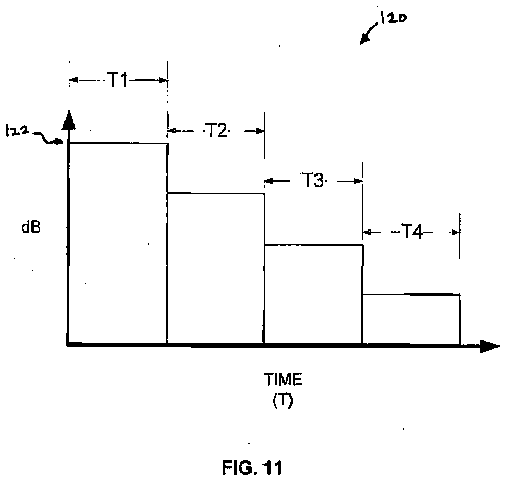

In yet another variation shown in the plot **120** of Fig. 11, the transducer **66** may be programmed to gradually decrease its amplification over a period of days, weeks, months, or even longer to facilitate the withdrawal of a user's dependence on the masking noise. For instance, the transducer **66** may initially provide a first dB level **122** for a first period of time **T1.** The assembly may then automatically reduce its amplification level over a second period of time **T2,** and eventually further reduce its amplification level over a third period of time **T3,** and another reduction over a fourth period of time **T4,** and so on, until the user no longer requires use of the device to tolerate his or her tinnitus condition. Each period of time may be programmed to range uniformly or otherwise anywhere from days to months or even longer depending upon the user's tinnitus condition.

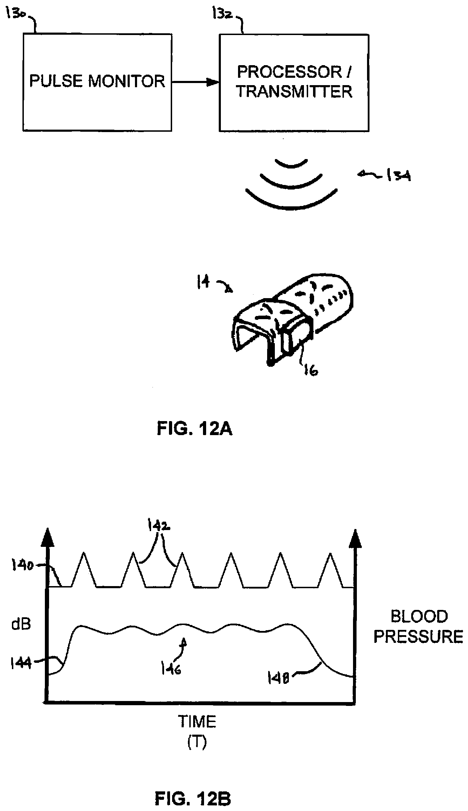

In yet another variation shown in Figs. 12A and 12B, conditions such as pulsatile tinnitus, as mentioned above, may also be treated using the hearing aid and/or sound generating assembly **14.** In patients suffering from pulsatile tinnitus, the sound associated with tinnitus typically occurs with a rhythmic pulsing sound attuned to the patient's heartbeat. Accordingly, the electronics and/or transducer assembly 16 within the assembly **14** may be programmed to produce a masking noise or to raise its amplification of ambient noise correspondingly with the patient's detected pulse. A pulse monitor **130** may be worn by the patient, e.g., around the wrist, and may be connected wirelessly or otherwise to processor/transmitter **132.** The detected pulse may be processed by processor **132** and transmitted wirelessly **134** to hearing aid and/or sound generating assembly **14,** as shown in Fig. 12A.

The received signals may be utilized by assembly **14** to raise its amplification dB level **144** and to maintain an elevated level **146** corresponding to the detected heart beat **142,** as seen along plot **140,** which corresponds to the detected heart beat, blood pressure, electrical activity, etc. of the patient's heart, as shown in Fig. 12B. After a preset or programmed period of time, as described above, the amplification level may be automatically decreased **148.**

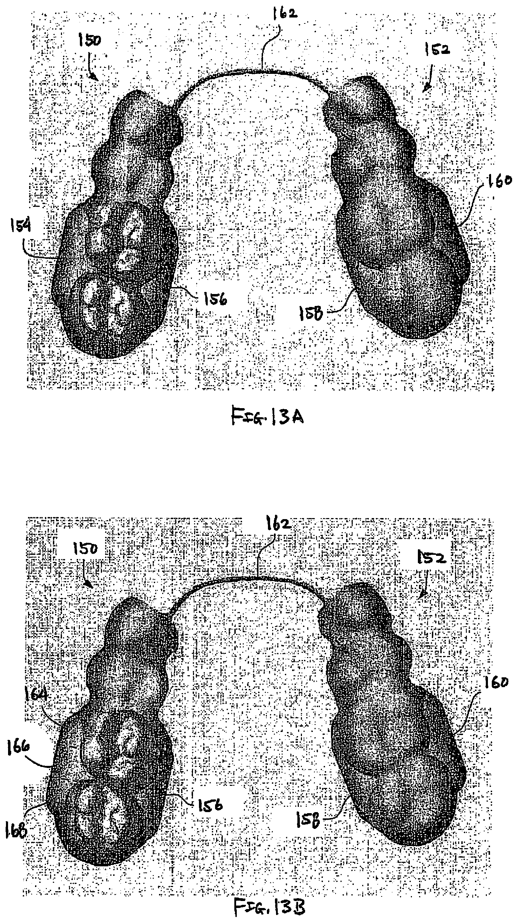

In yet another variation for delivering a tinnitus treatment to a patient, a configuration utilizing a connecting member **162** which may be positioned along the lingual or buccal surfaces of a patient's row of teeth to connect a first tooth retaining portion **150** and a second tooth retaining portion **152** is shown in Fig. 13A. One or more transducer assemblies **154, 156** may be integrated within the first retaining portion **150** to align along the buccal and lingual surfaces of one or more teeth. Similarly, one or more transducer assemblies **158, 160** may also be integrated within the second retaining portion **152** to align along the lingual and buccal surfaces of one or more teeth.

Fig. 13B illustrates a variation where at least one of the transducer assemblies integrated with a retaining portion **150** may be configured to include a battery **164,** transducer **166,** and associated electronics **168** such as a receiver, processor, and memory. The battery **164** may be a rechargeable or disposable type battery and the transducer **166** may be an electromagnetic transducer or a piezoelectric transducer. Piezoelectric transducers in particular may be used in various configurations due in part to the various vibrational modes which may be utilized to transmit the acoustic signals as vibrations through a tooth or teeth. Any number of transducers may be utilized for particular applications. For instance, low voltage multi-layer piezoelectric transducers manufactured by Morgan Electro Ceramics Ltd. (Wrexham, England) may be utilized for the applications described herein. Further examples of transducers and housing assemblies which may be utilized for the tinnitus treatments herein are shown and described in further detail in U.S. Pat. Apps. 11/741,648 filed April 27, 2007 and 11/754,823 filed May 29, 2007.

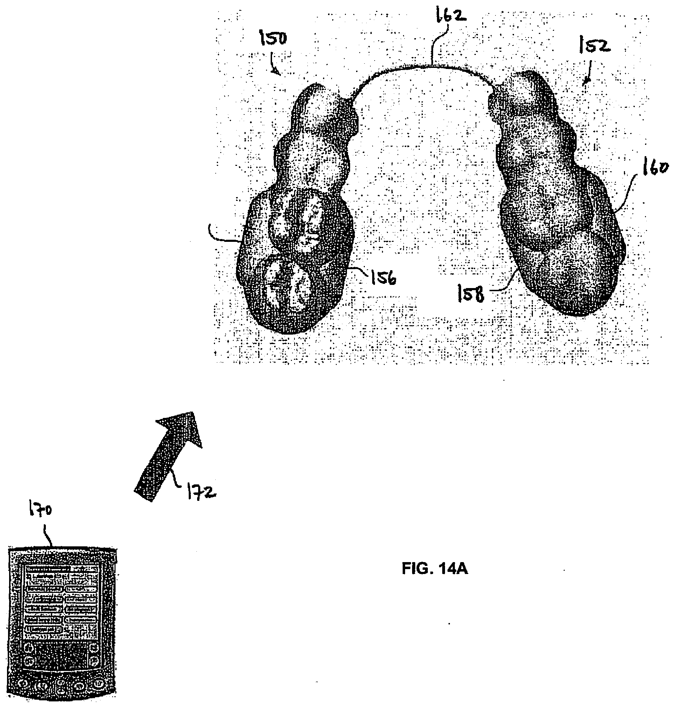

The system for tinnitus treatment can take a number of different forms. In one example, an external programming device **170** such as a PDA, cell phone, music player, etc. can be programmed to transmit **172,** e.g., via RF or Blue Tooth® (BLUETOOTH SIG, INC., Bellevue, WA), etc. the sound therapy programming treatment algorithm to the retainer **150,** as shown in Fig. 14A. For transmitting signals **172** from external device **170** to retainer **150,** particularly when utilizing RF, the signals **172** may be transmitted in the range of 1 MHz to 6 GHz. As described above for Fig. 8B, external device **170** may be programmed by the clinician for specific algorithms of treatment with one or more programs (for sleep, work, during exercise, etc.) that the patient or physician can select via the device **170.**

Additionally, the patient may control certain features of the external device **170** for enhanced comfort or additional programming features. For instance, the patient may control an ON/OFF selection, a volume of the treatment signal, program an alarm feature such that the treatment begins and/or ends at preselected times, program a sleep feature where the patient can program the retainer **150** to activate for a predetermined length of time before automatically shutting off, select desired sound files, etc. The external device **170** may also be programmed to upload selected files, retain a compliance indicator or data log of the times and duration which the patient used the retainer **150,** and it may also utilize a power indicator to notify the user that either the external device **170** and/or retainer **150** is powered.

The physician can also lock the patient from making any adjustments in program choice or volume of the tinnitus treatment. In either case, the external device **170** may upload the selected treatment to the retainer **150** and download compliance information for storage for the physician to review for further treatment enhancement, if necessary. Moreover, external device **170** may carry its own battery power supply which may be recharged periodically, as described above, or simply re-supplied with a new power supply.

To maintain consistency and uniformity with industry standards, the external device **170** may be programmed to conform with NOAH, which is an industry standard supported by a framework of companies within the audiology industry. Adherence to this industrial standard may allow for the programming information as well as any audiological measurements to interface with a common database.

Other tinnitus treatment algorithms which utilize software to spectrally modify audio signals in accordance with predetermined masking algorithms which modify the intensity of the audio signal at selected frequencies may also be used. For instance, a masking algorithm may provide for intermittent masking of the tinnitus where, at listening levels, during peaks of the audio signal, such as music, the tinnitus is completely obscured while during troughs in the audio signal, the perception of tinnitus occasionally emerges. Such algorithms may be programmed and transmitted to the retainer **150** and transmitted via vibrational conductance to the patient's tooth or bone. Details of such algorithms are described in further detail in U.S. Pat. Pub. 2004/0141624 A1 filed December 4, 2003.

A certain number of patients who suffer from tinnitus also suffer from hearing loss. Upwards of 80% of the patients with tinnitus also have some form of hearing loss which is a significant issue in treating the tinnitus with a sound therapy device that is meant to provide tinnitus therapy while also allowing the patient to continue with his/her normal daily activities. One approach to compensating for the hearing loss while also treating tinnitus includes a combination treatment **176** utilizing the oral appliance **150** for treating the tinnitus along with a hearing aid **174** for treating the hearing loss. Oral appliance **150** may also compensate for the sensorineural hearing loss by increasing the tinnitus treatment signal itself by up to 40 dB for treating the tinnitus without increasing for the input hearing. Any tone, music, or treatment using a wide-band and or narrow band noise may also be calibrated and equalized to compensate for impedances of the tooth and bone as well as for the sensorineural hearing loss and then that sound may be generated via the actuatable transducer. Calibration and equalization can be done using several approaches. One approach is to use average impedance among a group of subjects representative of the targeted population. Another approach is to customize the calibration and equalization by obtaining the teeth and bone impedances for each patient.

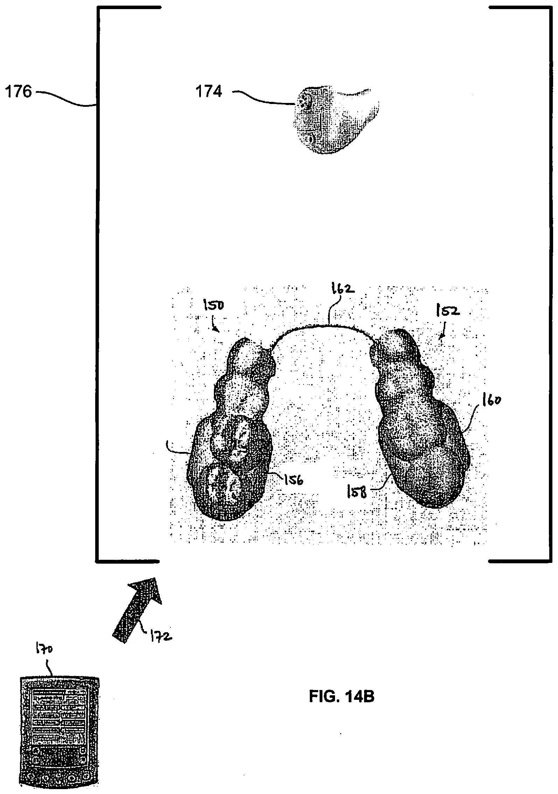

Most conventional hearing aid devices **174** are typically worn outside the ear or within the ear canal of the patient and does not allow for use of a tinnitus treatment device. However, use of the oral appliance **150** worn upon the user's tooth or teeth does not interfere with the wearing of a hearing aid within the ear but can instead complement the use of the hearing aid in conjunction with the appliance **150,** as shown in Fig. 14B.

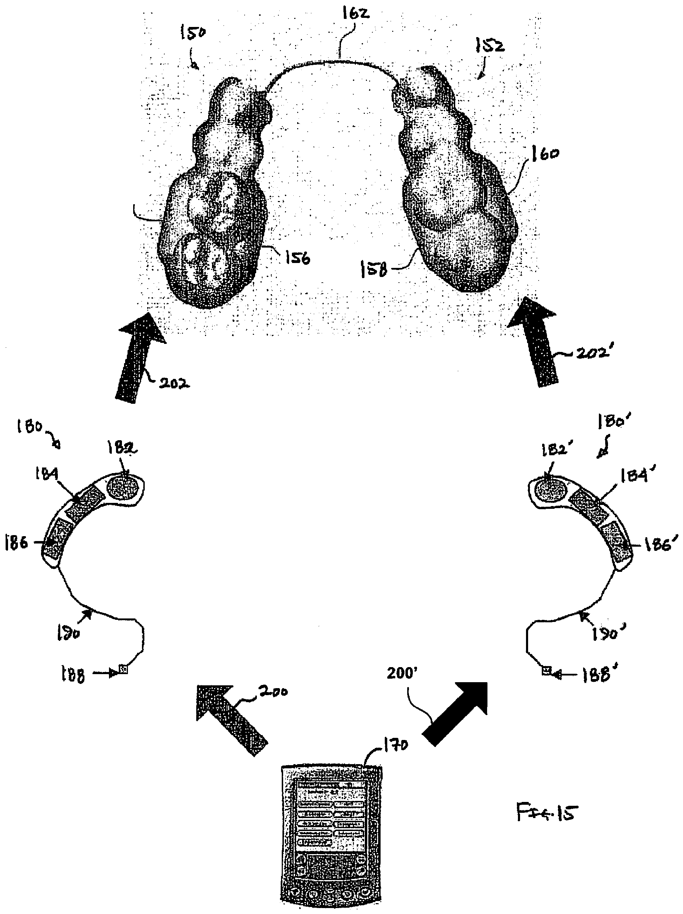

Another approach for treating patients having both tinnitus and hearing loss may include a combination therapy system 180 which may include a microphone **188** and receiver that would allow external sounds to be transmitted to the retainer device **150,** amplified and then subsequently delivered to the cochlea via bone conduction, as described above. The combination system **180** may include a connecting wire **190** which electrically couples microphone **188** to system **180** which may also include a processor **184** and a wireless transmitter **186** which may both be powered by battery **182,** as shown in Fig. 15. System **180** may amplify external sounds received by the user but it may also receive the tinnitus programming information **200** from an external device **170.** Processor **184** may combine any perceived auditory signals detected by microphone **188** and combine the tinnitus programming information **200** received from external device **170** and transmit this combined information **202** to retainer **150** for vibratory conduction to the user.

Although a single therapy system **180** may be used, a second complementary system **180'** also containing a microphone **188'** coupled by wire **190'** to a processor **184',** a wireless transmitter **186,** and battery **182'** may be utilized to also receive tinnitus programming information **200'** from external device **170** and to transmit **202'** any perceived auditory signals along with the tinnitus programming to the same or different retainer **160** for vibratory conduction to the user for treatment.

Additionally, in determining patient-specific frequency information, as described above, the assembly **16** can also be used to measure a patient's bone conductive hearing loss directly through the assembly **16** for determining appropriate gain levels for a patient's individual hearing loss profile. To measure the conductive hearing loss, the assembly **16** can be connected, wirelessly or wired, to a standard audiometer to measure the hearing threshold of the patient at the tooth or bone directly through assembly **16** by using standard audiometric protocols.

The patient may be asked to match their tinnitus sound level and frequency by listening to tones at different frequencies generated through the assembly **16** to first establish the tinnitus frequency. The frequency level may be gradually increased to match the tinnitus level perceived by the patient. With this correlated information, the assembly **16** and/or external device **170** may be programmed accordingly with the patient's hearing loss profile and adjusted for appropriate gain at each frequency during tinnitus treatment. Additionally, the programming software may configure a customized sound therapy treatment according to the physician's preference (e.g., wide or narrow band noise, recorded sounds, etc) based upon the patient's tinnitus frequency and level.

The applications of the devices and methods discussed above are not limited to the treatment of tinnitus and/or hearing loss but may include any number of further treatment applications. Moreover, such devices and methods may be applied to other treatment sites within the body. Modification of the above-described assemblies and methods for carrying out the invention, combinations between different variations as practicable, and variations of aspects of the invention that are obvious to those of skill in the art are intended to be within the scope of the claims.

## Claims

1. A tinnitus treatment system, comprising:
a tinnitus treatment algorithm having one or more frequencies of tinnitus perceived by a patient;
a housing configured to fit to at least a portion of at least one tooth (12) of the patient;
an actuatable transducer (40, 56, 66) disposed within or upon the housing and configured to transmit vibrations to a surface of the at least one tooth, wherein the housing is secured to the at least one tooth; and
a processor (32, 54, 64, 94, 132, 184) configured to actuate the transducer against the surface of the at least one tooth at the one or more frequencies according to the tinnitus treatment algorithm.

2. The system of claim 1 wherein the tinnitus treatment algorithm is adapted to actuate the transducer at a first sound level.

3. The system of claim 2 wherein the tinnitus treatment algorithm is adapted to gradually reduce or increase the sound level linearly or non-linearly over a predetermined period of time.

4. The system of claim 2 wherein the tinnitus treatment algorithm is adapted to maintain the first sound level for a predetermined period of time.

5. The system of claim 2 wherein the tinnitus treatment algorithm is adapted to actuate the transducer at a second sound level lower than or greater than the first decibel level.

6. The system of claim 1 further comprising a hearing aid device for amplifying an external sound received by the patient.

7. The system of claim 1 further comprising an external transmission device configured to wirelessly transmit the tinnitus treatment algorithm to the processor for actuating the transducer.

8. The system of claim 1 further comprising a microphone in electrical communication with the processor and configured to receive auditory signals.

9. A tinnitus treatment system, comprising:
a tinnitus treatment algorithm having one or more frequencies of tinnitus perceived by a patient;
a housing configured to fit to at least a portion of at least one tooth (12) of the patient
an actuatable transducer (40, 56, 66) disposed within or upon the housing and configured to transmit vibrations to a bone structure of the patient, wherein the housing is secured to the at least one tooth; and
a processor (32, 54, 64, 94, 132, 184) configured to actuate the transducer against the bone structure at the one or more frequencies according to the tinnitus treatment algorithm.

10. The system of claim 9 wherein the tinnitus treatment algorithm is adapted to actuate the transducer at a first sound level.

11. The system of claim 9 further comprising a hearing aid device for amplifying an external sound received by the patient.

12. The system of claim 9 further comprising an external transmission device configured to wirelessly transmit the tinnitus treatment algorithm to the processor for actuating the transducer.

13. The system of claim 9 wherein the housing is configured for placement against a tooth or teeth of the patient.

14. The system of claim 1 wherein the actuatable transducer (40, 56, 66) is positioned along a side surface of the housing, and wherein the actuatable transducer (40, 56, 66) is aligned along a buccal or lingual surface of the at least one tooth.

15. The system of claim 14 wherein the surfaces comprise buccal or lingual surfaces of the at least one tooth.

## Patentansprüche

1. Tinnitusbehandlungssystem, umfassend:
einen Tinnitusbehandlungsalgorithmus, der eine oder mehrere Frequenzen von Tinnitus hat, die von einem Patienten wahrgenommen werden;
ein Gehäuse, das konfiguriert ist, zu mindestens einem Abschnitt von mindestens einem Zahn (12) des Patienten zu passen;
einen betätigbaren Wandler (40, 56, 66), der sich innerhalb oder auf dem Gehäuse befindet und konfiguriert ist, Schwingungen auf eine Oberfläche des mindestens einen Zahns zu übertragen, wobei das Gehäuse an dem mindestens einen Zahn befestigt ist; und
einen Prozessor (32, 54, 64, 94, 132, 184), der konfiguriert ist, den Wandler gegen die Oberfläche des mindestens einen Zahns mit der einen oder den mehreren Frequenzen gemäß dem Tinnitusbehandlungsalgorithmus zu betätigen.

2. System nach Anspruch 1, wobei der Tinnitusbehandlungsalgorithmus angepasst ist, den Wandler bei einem ersten Geräuschpegel zu betätigen.

3. System nach Anspruch 2, wobei der Tinnitusbehandlungsalgorithmus angepasst ist, den Geräuschpegel linear oder nichtlinear über eine vorbestimmte Zeitdauer zu verringern oder zu erhöhen.

4. System nach Anspruch 2, wobei der Tinnitusbehandlungsalgorithmus angepasst ist, den ersten Geräuschpegel über eine vorbestimmte Zeitdauer zu erhalten.

5. System nach Anspruch 2, wobei der Tinnitusbehandlungsalgorithmus angepasst ist, den Wandler bei einem zweiten Geräuschpegel zu betätigen, der niedriger ist oder höher ist als der erste Dezibelwert.

6. System nach Anspruch 1, ferner umfassend eine Hörhilfevorrichtung zum Verstärken eines Außengeräusches, das von dem Patienten empfangen wird.

7. System nach Anspruch 1, ferner umfassend eine externe Übertragungsvorrichtung, die konfiguriert ist, dem Prozessor den Tinnitusbehandlungsalgorithmus zum Betätigen des Wandlers drahtlos zu übertragen.

8. System nach Anspruch 1, ferner umfassend ein Mikrofon in elektrischer Verbindung mit dem Prozessor und dazu konfiguriert, akustische Signale zu empfangen.

9. Tinnitusbehandlungssystem, umfassend:
einen Tinnitusbehandlungsalgorithmus, der eine oder mehrere Frequenzen von Tinnitus hat, die von einem Patienten wahrgenommen werden;
ein Gehäuse, das konfiguriert ist, zu mindestens einem Abschnitt von mindestens einem Zahn (12) des Patienten zu passen;
einen betätigbaren Wandler (40, 56, 66), der sich innerhalb oder auf dem Gehäuse befindet und konfiguriert ist, Schwingungen auf eine Knochenstruktur des Patienten zu übertragen, wobei das Gehäuse an dem mindestens einen Zahn befestigt ist; und
einen Prozessor (32, 54, 64, 94, 132, 184), der konfiguriert ist, den Wandler gegen die Knochenstruktur mit der einen oder den mehreren Frequenzen gemäß dem Tinnitusbehandlungsalgorithmus zu betätigen.

10. System nach Anspruch 9, wobei der Tinnitusbehandlungsalgorithmus angepasst ist, den Wandler bei einem ersten Geräuschpegel zu betätigen.

11. System nach Anspruch 9, ferner umfassend eine Hörhilfevorrichtung zum Verstärken eines Außengeräusches, das von dem Patienten empfangen wird.

12. System nach Anspruch 9, ferner umfassend eine externe Übertragungsvorrichtung, die konfiguriert ist, dem Prozessor den Tinnitusbehandlungsalgorithmus zum Betätigen des Wandlers drahtlos zu übertragen.

13. System nach Anspruch 9, wobei das Gehäuse zum Platzieren gegen einen Zahn oder Zähne des Patienten konfiguriert ist.

14. System nach Anspruch 1, wobei der betätigbare Wandler (40, 56, 66) entlang einer Seitenfläche des Gehäuses angeordnet ist und wobei der betätigbare Wandler (40, 56, 66) entlang einer bukkalen oder lingualen Oberfläche des mindestens einen Zahns ausgerichtet ist.

15. System nach Anspruch 14, wobei die Oberflächen bukkale oder linguale Oberflächen des mindestens einen Zahns umfassen.

## Revendications

1. Système de traitement d'acouphène, comprenant :
un algorithme de traitement d'acouphène possédant une ou plusieurs fréquences d'acouphène perçues par un patient ;
un boîtier configuré pour aller sur au moins une portion d'au moins une dent (12) du patient ;
un transducteur actionnable (40, 56, 66) disposé à l'intérieur du, ou sur le, boîtier et configuré pour transmettre des vibrations à une surface de l'au moins une dent, dans lequel le boîtier est fixé à l'au moins une dent ; et
un processeur (32, 54, 64, 94, 132, 184) configuré pour actionner le transducteur contre la surface de l'au moins une dent aux une ou plusieurs fréquences selon l'algorithme de traitement d'acouphène.

2. Système selon la revendication 1, dans lequel l'algorithme de traitement d'acouphène est adapté pour actionner le transducteur à un premier niveau sonore.

3. Système selon la revendication 2, dans lequel l'algorithme de traitement d'acouphène est adapté pour progressivement réduire ou augmenter le niveau sonore linéairement ou non linéairement durant une période prédéterminée.

4. Système selon la revendication 2, dans lequel l'algorithme de traitement d'acouphène est adapté pour maintenir le premier niveau sonore pendant une période prédéterminée.

5. Système selon la revendication 2, dans lequel l'algorithme de traitement d'acouphène est adapté pour actionner le transducteur à un second niveau sonore inférieur ou supérieur au premier niveau de décibel.

6. Système selon la revendication 1, comprenant en outre un dispositif d'aide auditive pour amplifier un son externe reçu par le patient.

7. Système selon la revendication 1, comprenant en outre un dispositif de transmission externe configuré pour transmettre sans fil l'algorithme de traitement d'acouphène au processeur pour actionner le transducteur.

8. Système selon la revendication 1, comprenant en outre un microphone en communication électrique avec le processeur et configuré pour recevoir des signaux auditifs.

9. Système de traitement d'acouphène, comprenant :
un algorithme de traitement d'acouphène possédant une ou plusieurs fréquences d'acouphène perçues par un patient ;
un boîtier configuré pour aller sur au moins une portion d'au moins une dent (12) du patient ;
un transducteur actionnable (40, 56, 66) disposé à l'intérieur du, ou sur le, boîtier et configuré pour transmettre des vibrations à une structure osseuse du patient, dans lequel le boîtier est fixé à l'au moins une dent ; et
un processeur (32, 54, 64, 94, 132, 184) configuré pour actionner le transducteur contre la structure osseuse aux une ou plusieurs fréquences selon l'algorithme de traitement d'acouphène.

10. Système selon la revendication 9, dans lequel l'algorithme de traitement d'acouphène est adapté pour actionner le transducteur à un premier niveau sonore.

11. Système selon la revendication 9, comprenant en outre un dispositif d'aide auditive pour amplifier un son externe reçu par le patient.

12. Système selon la revendication 9, comprenant en outre un dispositif de transmission externe configuré pour transmettre sans fil l'algorithme de traitement d'acouphène au processeur pour actionner le transducteur.

13. Système selon la revendication 9, dans lequel le boîtier est configuré pour le placement contre une dent ou des dents du patient.

14. Système selon la revendication 1, dans lequel le transducteur actionnable (40, 56, 66) est positionné le long d'une surface latérale du boîtier, et dans lequel le transducteur actionnable (40, 56, 66) est aligné le long d'une surface buccale ou linguale de l'au moins une dent.

15. Système selon la revendication 14, dans lequel les surfaces comprennent des surfaces buccales ou linguales de l'au moins une dent.
